# EUROPEAN PATENT APPLICATION

(11) **EP 4 074 310 A1**
(43) Date of publication of application: **19.10.2022**
(21) Application number: 20927055.2
(22) Date of filing: 19.11.2020
(51) Int. Cl.: A61K 31/115, A61K 9/08, A61P 31/14

(54) **DRUG FOR TREATING CORONAVIRAL AND RETROVIRAL INFECTIONS AND HEPATITIS C**

(30) Priority: 26.03.2020 RU 2020112322
(71) Applicant: Laskavyi, Vladislav Nikolaevich, Saratov, 410018 (RU)
(72) Inventor: LASKAVYI, Vladislav Nikolaevichul., Saratov, 410018 (RU); SHURDOV, Mikhail Arkadevich., Moskva 119049 (RU)
(74) Representative: Spengler, Robert
(86) International application number: PCT/RU2020/050334
(87) International publication number: WO 2021/194375

(57) **Abstract**

The invention relates to medicine and veterinary medicine, and more specifically to pharmacology, and can be used to treat viral infections caused by RNA viruses that have a lipid envelope, in particular coronaviral infections, AIDS and hepatitis C. The invention extends the range of drugs for the claimed purpose. The technical result is the creation of a drug having an intracellular, antiseptic effect that activates the production of endogenous formaldehyde in the body of a human and animals without having side effects and toxicity. The technical result is achieved by the use of an immunomodulating drug comprising 0.073-0.075% of formaldehyde in an isotonic solution of sodium chloride as a drug for intramuscular injections with a single 5 ml injection dosage for treating coronaviral and retroviral infections and hepatitis C.

## Description

The invention relates to medicine and veterinary medicine, and more specifically to pharmacology, and can be used to treat viral infections caused by lipid-enveloped RNA viruses, in particular, coronavirus infections, AIDS, and hepatitis C. The invention expands the arsenal of means for the claimed purpose.

A remedy to treat hepatitis C is known (see RF patent No. 2,244,554, class IPC A61K35/78, publ. 20.01.2005), containing a birch bark extract with a betulin content of more than 70% and a pharmaceutically acceptable carrier. The remedy is administered orally to the patient.

Since the remedy is an herbal preparation, it is a multicomponent system that could not only stimulate protection against viruses, but also change the homeostasis of the human and animal body, causing allergic reactions to its components in potential allergy sufferers, pregnant and lactating women. It should be noted that the interaction of betulin with other drugs has not been established, however, it is not excluded.

A composition is known from a highly purified yeast RNA extract, having at least 75% of fragments of 25 ± 10 nucleotides by weight, with a purity preferably of at least 99%, in combination with mannitol in a weight proportion of 2:1 to 3:1 (see RF patent No. 2,597,150, class IPC A61K31/7105, published on September 10, 2016), while the yeast RNA extract makes up at least 50% of the weight of the composition. The composition is intended for the treatment of viral diseases caused by viruses of the Orthomyxoviridae, Paramyxovirus, Hepatitis, Herpesviridae families, as well as enteroviruses and adenoviruses. In addition, modified yeast RNA is able to inhibit the reproduction of the viruses of influenza, hepatitis C, genital herpes, human immunodeficiency, and Coxsackie B.

However, this drug cannot completely eliminate all of these viruses, but can only reduce the viral load, for example, in viral hepatitis C. In addition, such a serious interference into RNA cannot but affect the homeostasis of the body.

A composition is known for the treatment of severe forms of viral infections in the form of tablets (see RF patent 2,559,179, class IPC A61K9 / 10, publ. 08/10/2015), containing recombinant interferon selected from the group: recombinant interferon-alpha, recombinant interferon-beta, recombinant interferon-gamma; antioxidants selected from the group: mexidol, emoxipin, dibunol, alpha-lipoic acid, carnitine chloride; amino acids selected from the group: acetylcysteine, cysteine, lysine, arginine; anabolic action regenerants selected from the group: potassium orotate, riboxin, methyluracil, as well as a shaping base.

However, there are limitations when using such a compound preparation. The drug should be used with caution in liver disease, severe depressive illness and/or a history of suicidal thoughts, epilepsy and pregnancy. Caution must be exercised in the treatment with this drug of patients receiving anticonvulsants, patients with heart failure stage III-IV according to the NYHA classification and in patients with cardiomyopathy, bone marrow dysfunction, anemia or thrombocytopenia.

An antiseptic against influenza is known (see RF patent No. 2,355,391, class IPC A61K31 / 045, published on May 20, 2009), containing a cationic surfactant, low-molecular-weight glycol or glycerol, and ammonium salt and constituent, urea, and additionally ammonium nitrate as an ammonium salt, at a certain ratio of the components. Dodecyldimethylammonium bromide or dodecyldimethylammonium chloride is used as the cationic surfactant. Triethylene glycol, or 1,2-propylene glycol, or polypropylene glycol is used as a low-molecular-weight glycol. Water or 8-20% aqueous solution of ethyl or isopropyl alcohol is used as a constituent. The invention provides an increase in the biocidal activity of the target product during long-term storage, expansion of the action spectrum, reduction of irritating properties and toxicity.

However, this drug can only be used to kill viruses which are outside the body. And this drug is not supposed to treat viral diseases.

Also known is an agent for the prevention and treatment of viral infections caused by RNA-containing viruses, preferably influenza A viruses or rhinoviruses (see RF patent No. 2,524,304, class IPC A61M15/00, publ. 27.07.2014). The agent contains, as its active part, a salt of o-acetylsalicylic acid, and, as a target additive, an amino acid selected from the group including lysine, arginine, ornithine, and diaminobutyric acid. The agent is intended for aerosol administration by inhalation through the nose or mouth.

The main disadvantage of this drug is that the use of acetylsalicylic acid may cause severe irritation of the respiratory tract. And even a decrease in this possibility with the help of a complex of a salt of o-acetylsalicylic acid with an amino acid does not completely exclude the possibility of allergic reactions.

A high risk when using this composition exists in people with a history of urticaria, rhinitis caused by taking acetylsalicylic acid and other NSAIDs, hemophilia, hemorrhagic diathesis, hypoprothrombinemia, dissecting aortic aneurysm, portal hypertension, vitamin K deficiency, hepatic and/or renal insufficiency, deficiency of glucose-6-phosphate dehydrogenase, Reye's syndrome, children's age (up to 15 years - the risk of developing Reye's syndrome in children with hyperthermia on the background of viral diseases), hypersensitivity to acetylsalicylic acid and other salicylates.

Acetylsalicylic acid, even in small doses, reduces the excretion of uric acid from the body, which may cause an acute attack of gout in predisposed patients.

It should be noted that acetylsalicylic acid often interacts with other drugs, for example, with calcium channel blockers, drugs to limit the intake of calcium or to increase the excretion of calcium from the body; or the efficiency of diuretics (spironolactone, furosemide, etc.) decreases.

Closest to the claimed is the immunomodulatory agent containing an active principle and targeted additives (see RF patent No. 2,077,882, class IPC A61K 31/115, publ. 27.04.1997). As the active principle, it contains formaldehyde, and NaCl and distilled water as target additives, while it is an injection solution containing, wt.%: formaldehyde 0.07-0.24, NaCl 0.9-0.95, distilled water - the rest up to 100%.

These analogs show that the design of antiviral agents is far from a solved problem. Therefore, searching for new means of protection against viral infections is very relevant.

The technical problem of the claimed invention is the design of an effective injectable for the treatment of infections caused by RNA-containing viruses in humans and animals and the expansion of the arsenal of pharmaceuticals against coronavirus, retroviral infections and hepatitis C.

The technical result is the design of an agent with an intracellular antiseptic effect, which activates the production of endogenous formic aldehyde in the human and animal body in the absence of side effects and toxic effects.

The technical result is achieved by using an immunomodulating agent containing formic aldehyde in an amount of 0.073-0.075% in isotonic sodium chloride solution as an agent for intramuscular injections with a single dose of 5 mL per injection for the treatment of coronavirus, retroviral infections and hepatitis C. Depending on the severity of the disease, the agent is administered according to various schemes, namely: either 1 time per day for 7 days, or twice, with the second time or on the 7^{th} day after the first injection or a month after the first injection. The agent can be administered on the 2^{nd}, 3^{rd} and 10^{th} day after the first injection. In particularly severe cases, the agent is administered 3 times with an interval between each injection of 12 hours, then after one or two days it is administered 5 more times with an interval between each injection of 12 hours, then one day after the last injection, the agent is administered once, then after three days a second single injection is carried out, after which, five days after the last injection, a final single administration of the agent is carried out.

Sources of patent and scientific and technical information known to the authors describe no means for the treatment of viral infections caused by RNA-containing viruses, in particular, coronavirus infections, AIDS, hepatitis C in humans and animals, based on the activation of the production of intracellular endogenous formic aldehyde. Its new function and proposed purpose do not clearly follow from its previously known properties. Methods of administration of the drug have not been previously described anywhere.

Currently, there is no universal antiseptic drug which could destroy the pathogens of infectious diseases (viruses) inside the cells of the human and animal body without negatively affecting homeostasis. Existing antiseptic agents are used, as a rule, to inactivate pathogens in the external environment.

Of great interest is formic aldehyde (formaldehyde), one of the simplest compounds in nature. This substance is classified as an aseptic drug of a chemical nature (organic compounds) for the destruction of pathogens of infectious diseases in the external environment, as a chemical preservative for cells and tissues in histology and pathological anatomy.

It has now been established that there is endogenous formaldehyde in living organisms, which is a natural metabolite and is involved in many biochemical reactions of the body. The concentration of endogenous formaldehyde may reach 0.2-0.5 mM, and its content in the blood may be 0.05-0.1 mM (M. Casanova, H. D. Heck, J. I. Everitt, W. W. Harrington, J. A. Popp. Formaldehyde concentrations in the blood of Rhesus monkeys after inhalation exposure // Food Chem. Toxicol. Int. J. Publ. Br. Ind. Biol. Res. Assoc. 1988, 26,715; X. Song, X. Han, F. Yu, J. Zhang, L. Chen, C. Lv, A reversible fluorescent probe based on C=N isomerization for the selective detection of formaldehyde in living cells and in vivo.//The Analyst 2018,143, 429; Human Endogenous Formaldehyde as an Anticancer Metabolite: Its Oxidation Downregulation May Be a Means of Improving Therapy Yuri L. Dorokhov Ekaterina V. Sheshukova Tatiana E. Bialik Tatiana V. Komarova //BioEssays 2018, 1800136). Formaldehyde is known to easily penetrate into all cells and to be easily removed when its concentration exceeds its physiological norm. The physiological level of formaldehyde in the body is constantly maintained due to the continuous action of cellular enzymes which oxidize formaldehyde in three separate pathways involving P450 monooxygenases, mitochondrial AlDH2 and the gene encoding ADH5 or formaldehyde dehydrogenase (Dorokhov Y. L. Metabolic methanol: molecular pathways and physiological role //Physiol. Rev. 2015. 95(2), 603-644; Yuri L. Dorokhov Ekaterina V. Sheshukova Tatiana E. Bialik Tatiana V. Komarova Human Endogenous Formaldehyde as an Anticancer Metabolite: Its Oxidation Downregulation May Be a Means of Improving Therapy //BioEssays 2018, 1800136). Formaldehyde is formed in the liver under the action of microsomal dimethylase, during the biotransformation of dihalogen derivatives of methane and methyl methacrylate (Malyutina N.N., Taranenko L.A. Pathophysiological and clinical aspects of the effects of methanol and formaldehyde on the human body // Modern problems of science and education. - 2014. - No. 2 [Russ.]; URL: http://science-education.ru/ru/article/view?id=12826 (date of access: 17.03.2020)). Formaldehyde is used by the body for the synthesis of thymidine, purine and other acids; it is formed during the destruction of serine and, to a lesser extent, other amino acids. When it enters the blood through a series of enzymatic transformations, it is oxidized in the liver to formic acid, while methyl alcohol is formed in the liver. Further, formic acid is metabolized under the action of formate dehydrogenase to CO₂ or is involved in the tetrahydrofolic acid system in the exchange of one-carbon residues. Formaldehyde easily interacts with proteins, amines, amides, nucleoproteins, and nucleic acids (Malyutina N.N., Taranenko L.A. Pathophysiological and clinical aspects of the effects of methanol and formaldehyde on the human body // Modern problems of science and education. - 2014. - No. 2 [Russ.]; URL: http://science-education.ru/ru/article/view?id=12826 (date of access: 17.03.2020)).

It is found in all internal fluids of humans and animals in small quantities, and only in the urine it is 20% more than, for example, in the blood, which indicates the main way it is excreted from the body.

Studies have shown that low formaldehyde concentrations (e.g. 10 mmol/L) promote HeLa cell proliferation. However, when the formaldehyde concentration reaches 62.5 mmol/L, it inhibits cell growth.

A certain concentration of formaldehyde in the body is important for the vital activity of cells, since endogenous formaldehyde is an important metabolite, however, in the case of a strong increase in its concentration, it may also act as a genotoxin (Richard J. Hopkinson, Christopher J. Schofield. Deciphering Functions of Intracellular Formaldehyde: Linking Cancer and Aldehyde Metabolism// Biochemistry 2018, 57, 6, 904-906).

Using the example of the porcine transmissible gastroenteritis virus, which belongs to coronaviruses, it was found that formalin inactivates the virus at a concentration of 0.01% (Stepanek J., Mensik J., Pospisil Z.,Rozkony V. Vlev formalinu a betapropiolaktonuna infekcnost a antigenicitu puvodce virovegastroenterit du prasat (VGR). // Veter. Med. (CSSR). - 1973. - V. 18. - #5. - P. 269-280). Under the influence of formalin at 37°C, this virus loses its pathogenicity just after 150 min (Bohl E.H. Transmissible gastroenteritis virus (classical enteric variant). // Virus Infec. Procines. Amsterdam ect. - 1989. - P. 139-153). Other coronaviruses (viruses of hepatitis of mice, infectious bronchitis of chickens, infectious peritonitis of cats, viruses of gastroenteritis and respiratory infections in cattle, human gastroenteritis, etc.) are also sensitive to formaldehyde.

The developed agent is a solution of formic aldehyde in an isotonic sodium chloride solution, obtained using common preparation methods, the active component is dissolved in the carrier in an ultra-low dose and the concentration used causes no toxic effect in normal cells.

The agent has a complex effect on the body and helps maintain homeostasis. It belongs to a new class of drugs which promote the activation of an antigen-specific immune response, whose development achieves the complete elimination of the infectious agent from the body.

In our opinion, parenteral administration of exogenous formic aldehyde (formaldehyde) would lead to an increase in its concentration in human lymph and blood. This, in turn, disrupts the outflow of formaldehyde from the cells of the body into the intercellular space, which leads to an increase in its amount inside the cells in accordance with one of Le Chatelier-Brown's rules, which states that with an increase in the concentration of one of the reaction products, the equilibrium shifts towards the formation of the initial materials (leftwards). Due to this, the inactivation of viruses inside the cells occurs.

An aqueous solution of formic aldehyde is a clear, colorless liquid with a peculiar pungent odor, miscible with water and alcohol in all proportions.

Formic aldehyde (HCOH) is a representative of the class of aldehydes. It is a colorless gas with a pungent odor, the molar weight of 30.03, its density at 20°C is 0.815, melting point 92°C, boiling point 19.2°C. It is well dissolve in water, alcohol.

Isotonic sodium chloride solution for injection is a colorless transparent liquid of a salty taste with a sodium chloride concentration of 0.85-0.95%. The solution is sterile, non-pyrogenic.

Sodium chloride is cubic crystals or white crystalline powder of salty taste, odorless. It is soluble in water (1:3).

The claimed agent is a clear, colorless, odorless liquid with a slightly salty taste. The agent is prepared as follows.

Take 2 parts (by weight) of a 36.5-37.5% medical solution of formic aldehyde, add it to 998 parts (by weight) of a sterile 0.85-0.95% sodium chloride solution for injection until the concentration of formic aldehyde in the agent is 0.073- 0.075%. The product is stored in a dark place at temperatures within 15-35°C.

**Example 1.** Take 0.2 mL of a 37% medical solution of formic aldehyde, add it to 99.8 ml of a sterile 0.9% (or 0.95%) isotonic sodium chloride solution. The mixture of solutions is thoroughly stirred. The final concentration of formic aldehyde in the resulting product will be 0.074 wt.%.

**Example 2.** Take 0.2 mL of a 36.5% medical solution of formic aldehyde, add it to 99.8 ml of a sterile 0.9% (or 0.95%) isotonic sodium chloride solution. The mixture of solutions is thoroughly stirred. The final concentration of formic aldehyde in the resulting product will be 0.073 wt.%.

### Example 3.

The ability of exogenous formaldehyde to activate the production of endogenous formaldehyde was studied by incubating cells with 14C-labeled formaldehyde. Labeled formaldehyde was used with a specific activity of 1.7 TBq/M and a volumetric activity of 10.1 GBq/L (5.9 mM solution) and a radiochemical purity of 98%. Fresh 37% formaldehyde (Sigma, USA) was used to obtain solutions with the required concentration.

Cell samples which had not been incubated with radiolabeled formaldehyde were used as a negative control.

The radioactivity of the samples was measured on a Tri-Carb 2800 TR β-counter (PerkinElmer, USA) in an ULTIMA GOLD LLT scintillator (Perkin Elmer, USA). An aliquot of the sample (0.1 mL) was thoroughly mixed with the scintillator (0.9 mL) and the radioactivity was measured within no more than 2 h after the mixture was prepared.

Cells were cultured on the surface of 48-well polystyrene plates.

Primary human gingival fibroblasts and cervical adenocarcinoma HeLa cells were used as cell cultures. Cells were cultured in IMDM culture medium supplemented with 10% fetal bovine serum and an antibiotic (penicillin and streptomycin) in an atmosphere of 6% CO₂, at 37°C.

During the experiment, the cells were placed into 48-well polystyrene plates with a well diameter of 1 cm, in the amount of 12 thousand cells per well, in triplicate for each type of sample (in 0.5 ml of culture medium). After 24 h of incubation after seeding the cells, the culture medium was removed, the cells were rinsed with 0.5 mL of physiological saline, and physiological saline without or with formaldehyde was added to the control and experimental wells and incubated with the cells for 30 min, 1 and 2 h (the final concentration 0.11%, 0.074%, 0.014%, 0.0028%, and 0.00056%, five-fold dilutions, 10,000 ppm/well). To study the effect of formaldehyde in the culture medium, 50 µl (1/10 volume) of a formaldehyde solution in physiological saline was added to the control and experimental wells with 450 µl of the culture medium (the concentration of formalin was 10 times higher than mentioned earlier, as a result, formaldehyde solutions were obtained in the same concentration as in the case of cell incubation in saline). After incubation, two aliquots of 100 µl of the medium (doubles) were taken from the supernatants to measure the total introduced radioactivity, then the formaldehyde solutions were removed, the cells were washed twice with phosphate buffer and treated with 0.25% trypsin solution (Sigma, USA) in order to dissociate the cells from the culture plastic surfaces. The cell suspension was sedimented by centrifugation at 800g for 10 min, and aliquots were taken from the trypsin supernatant. The cell precipitation was resuspended in 220 µl of saline and 2 aliquots of 100 µl were taken to measure the radioactivity of the samples.

After obtaining data on the radioactivity of the samples, the percentage of formaldehyde in the fraction was calculeted based on the specific radioactivity and the initial volume of the sample. Formaldehyde concentrations in the fraction were analyzed based on the specific molar radioactivity of formaldehyde solutions.

**Table 1. Formaldehyde accumulation in cells. Incubation in saline.**

| Added formaldehyde concentration, % | Primary human gingival fibroblasts, % of what added^{∗} | | | HeLa cervical adenocarcinoma cells, % of what added^{∗} | | |
|---|---|---|---|---|---|---|
| | Superna tant^{∗∗} | Trypsin eluate | Cells | Superna tant^{∗∗} | Trypsin eluate | Cells |
| 0.11 | 100 | 3.71±0.45 | 1.73±0.2 3 | 100 | 2.41±0.11 | 1.85±0.2 5 |
| 0.074 | 100 | 5.16±0.85 | 2.12±0.3 3 | 100 | 3.32±0.27 | 2.75±0.1 8 |
| 0.014 | 100 | 3.41±0.44 | 3.11±0.2 7 | 100 | 3.44±0.19 | 2.65±0.1 1 |
| 0.0028 | 100 | 1.73±0.23 | 1.26±0.1 1 | 100 | 2.14±0.08 | 1.34±0.0 5 |
| 0.00056 | 100 | 0.45±0.03 | 0.53±0.0 4 | 100 | 0.57±0.04 | 0.62±0.0 4 |

| | | | | | | |
|---|---|---|---|---|---|---|
| ^{∗} the table shows the mean values ± error of the mean. ^{∗∗}the radioactivity of the supernatant is taken as 100% | | | | | | |

As can be seen from Table 1, incubation of cells with formaldehyde dissolved in saline at a concentration of 0.074% activates the production of endogenous formaldehyde in primary human gum fibroblasts and in adenocarcinoma of the cervix by 18.4% and 32%, respectively, compared with cell incubation in a 0.11% formaldehyde solution.

It should be noted that similar results were obtained when cells were incubated with formaldehyde diluted in the culture medium. E.g., incubation of cells with a 0.074% formaldehyde solution activates the production of endogenous formaldehyde on primary human gum fibroblasts and in adenocarcinoma of the cervix by 20.6% and 30.2%, respectively, compared with incubation of cells in a 0.11% formaldehyde solution (Table 2).

**Table 2. Formaldehyde accumulation in cells. Incubation in culture medium.**

| Added formaldehyde concentration , % | Primary human gingival fibroblasts, % of what added^{∗} | | | HeLa cervical adenocarcinoma cells, % of what added^{∗} | | |
|---|---|---|---|---|---|---|
| | Supernat ant^{∗∗} | Trypsin eluate | Cells | Supernat ant^{∗∗} | Trypsin eluate | Cells |
| 0.11 | 100 | 1.77±0.16 | 1.35±0.0 9 | 100 | 1.83±0.12 | 1.92±0.1 6 |
| 0.074 | 100 | 2.33±0.34 | 1.7±0.11 | 100 | 2.54±0.14 | 2.75±0.1 8 |
| 0.014 | 100 | 1.36±0.15 | 1.25±0.1 3 | 100 | 1.92±0.11 | 1.52±0.0 9 |
| 0.0028 | 100 | 0.56±0.16 | 0.55±0.0 7 | 100 | 0.45±0.06 | 0.64±0.1 3 |
| 0.00056 | 100 | 0.23±0.03 | 0.25±0.0 2 | 100 | 0.20±0.02 | 0.28±0.0 3 |

| | | | | | | |
|---|---|---|---|---|---|---|
| ^{∗}the table shows the mean values ± error of the mean. ^{∗∗}the radioactivity of the supernatant is taken as 100%. | | | | | | |

Thus, studies have shown that only the used 0.074% formaldehyde, compared with other concentrations (0.11%, 0.028%, 0.00052% and 0.014%), is able to activate the production of endogenous formaldehyde in the cells of primary human fibroblasts and adenocarcinoma of the cervix HeLa, which corresponds to Le Chatelier-Brown's rule. It states that with an increase in the concentration of one of the reaction products (in our case, exogenous formaldehyde at a concentration of 0.074%), the equilibrium shifts in the direction of increasing the formation of the initial (endogenous) formaldehyde in cells.

Below are examples of using the agent for various viral infections.

For therapeutic purposes, the agent is administered intramuscularly in 5 mL in one injection, and the administration schemes may be different. Namely:
- according to the first scheme, the agent is administered twice: on days 1 and 7 after the first injection;
- according to the second scheme, also twice, but with an interval of one month;
- according to the third scheme - on the 2nd, 3rd and 10th day after the first injection;
- according to the fourth scheme, the agent is administered 3 times with an interval between each injection of 12 h, then after 1-2 days it is administered 5 more times with an interval between each injection of 12 h. Then, one day after the last injection, the agent is administered once, then after three days a repeated single injection is carried out, after which the final single injection is carried out five days after the last injection; ad
- according to the fifth scheme - 1 time per day for seven days.

The number of injections in the protocols may be increased according to the disease severity.

**Example 4.** Coronavirus infection in pigs (porcine transmissible gastroenteritis) is recorded in sows, suckling piglets and weaned piglets.

In farms unfavorable for transmissible gastroenteritis, sows show a pulmonary form of the disease after farrowing, both pulmonary and intestinal forms are recorded in weaned piglets, and intestinal one are in suckling pigs.

An experiment was carried out on 16 sows-analogues with 153 newborn piglets in one of the queen cells, where transmissible gastroenteritis was recorded. To do this, a 0.074% solution of formic aldehyde was prepared. The first group of animals (8 sows with 75 newborn piglets) was injected with a 0.074% solution of formic aldehyde at the rate of 5 mL per sow and 2 mL per piglet. The control group (8 sows with 78 newborn piglets) was injected with a similar volume of saline. The agent was administered twice, namely: on the first and 7^{th} day after the first injection.

The animals were clinically observed up to two weeks of age, the clinical manifestation of transmissible gastroenteritis and the death of animals were recorded. The results obtained are presented in Table 3.

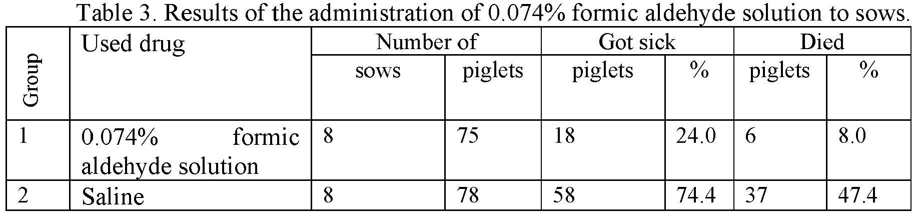

The results of our studies showed that the introduction of a 0.074% solution of formic aldehyde simultaneously to sows and newborn piglets significantly reduced the incidence and mortality of suckling pigs from transmissible gastroenteritis. This action in a two-link chain (sows and newborn piglets) must be considered from two perspectives. The first administration of the agent to sows provided a decrease in the number of pathogens in the body of mothers, reducing the dose of infection for newborns. The introduction of the drug to piglets provided a therapeutic effect.

As can be seen from the table, the incidence decreased by 50% (74.4-24.0), which was associated with a preventive effect to reduce the amount of the virus in mothers, and the mortality decreased by 6 times due to the therapeutic effect after the administration of the drug to piglets.

**Example 5.** Proof of the antiseptic effect of the agent on the example of a retroviral infection (AIDS). The studies were carried out on 6 volunteers.

The drug was administered to the first five patients at a dose of 5 mL on the 2^{nd}, 3^{rd} and 10^{th} day after the first injection, and the last (sixth) patient was administered the drug at a dose of 5 mL twice, and the second time - a month after the first introductions. Treatment results:
Patient 1, the initial viral load of 750,000 RNA/mL, the viral load two months after treatment was 2,187 RNA/mL, a decrease in viral load by 342 times was noted.
Patient 2, the initial viral load of 150,201 RNA/mL, the viral load two months after treatment was 1,230 RNA/mL, a decrease in viral load by 122 times was noted.
Patient 3, the initial viral load of 97,171 RNA/mL, the viral load two months after treatment was 1,101 RNA/mL, there was a decrease in viral load by 88 times.
Patient 4, the initial viral load of 78,122 RNA/mL, the viral load two months after treatment was 1,037 RNA/mL, a 75-fold decrease in viral load was noted.
Patient 5, the initial viral load of 78,122 RNA/mL, the viral load two months after treatment was 1,837 RNA/mL, a decrease in viral load by 42.5 times was noted.
Patient 6, the initial viral load of 11,182 RNA/mL, the viral load two months after treatment was 1,165 RNA/mL, a decrease in viral load by 9 times was noted.

All the patients showed a sharp decrease in viral load due to the antiseptic effect of the agent at the cellular level without a negative effect on the body and in the absence of a toxic effect.

**Example 6.** Proof of the antiseptic effect of the agent on the example of viral hepatitis C. The treatment was carried out on ten volunteers.

All the patients showed relapsing changes in viral replication, with the viral load fluctuating at 1 decimal log every 1-3 months (decreasing and rising periodically). In three patients, treatment was started in the seronegative phase of their disease.

Viral load minimization was observed in 7 patients, namely: the final level was 1,000 RNA copies/mL, while the initial level was from 2,500,000 RNA copies/mL.

Negative PCR was noted in 3 cases at the beginning of therapy in the seronegative phase of the disease.

All patients with viral hepatitis noted an improvement in their general condition, sleep, mood, increased efficiency, and better tolerated physical activity. The patients' social activity increased, their relationships in the family and at work improved.

**Example 7.** Patient M., 46 years old, SARS-CoV-2 coronavirus infection (based on anamnesis, clinical signs and radiographs).

Clinical signs: high temperature (38-39.2°C), unproductive persistent cough, respiratory failure with chest pain, malaise, and moderate headache.

X-ray pattern of the chest cavity: the presence of small foci of inflammation in the peripheral parts of the lung fields.

Treatment with antibiotics for three days did not work.

Treatment was carried out according to the following scheme. The agent was administered at a dose of 5 mL. Three injections of the agent every 12 h were conducted, then a day later five more injections of the agent every 12 h were carried out. Then one injection of the drug was administered a day later, another injection was administered three days later, and the final injection was administered five days after the previous injection.

Result: the clinical signs disappeared; there were no foci of inflammation on the radiograph. There were no side effects and toxic effects of the drug.

**Example 8.** Patient S., 35 years old - coronavirus infection (based on anamnesis and clinical signs).

Clinical signs: body temperature 37.2-37.7°C, unproductive persistent cough, malaise, and moderate headache. The patient had been in contact with people with a confirmed diagnosis of coronavirus infection. Treatment with antibiotics for 10 days did not work.

A course of treatment was carried out according to the following scheme: intramuscularly at a dose of 5 mL once a day for seven days.

Result: the clinical signs disappeared, there was a complete recovery. The absence of side effects and toxic effects of the drug was noted.

**Example 9.** Patient S., aged 56, was diagnosed with COVID-19 (PCR method).

Clinical signs at the start of treatment: body temperature 39.2°C, bilateral lung damage (25% on each side) according to computed tomography (CT), dry cough, chest pain, general weakness, sweating, does not smell. From the first day of the disease, he took antibiotics without a visible result.

On the 3^{rd} day of the disease, treatment with the claimed agent was started according to the following scheme: the agent was administered in 5 mL daily for 7 days with an interval of 24 h. Any antibiotics were no longer used.

### Result.

On the 2^{nd} day, the cough intensified and turned into a wet stage. The temperature dropped to 37.4°C, the headache disappeared. On the 3^{rd} day from the start of therapy, the temperature returned to normal (36.6°C), any pain symptoms disappeared. The general condition has improved. The patient noted a surge of strength, no sweating. On the 7^{th} day, the patient objectively recorded a complete recovery.

On the 14^{th} day after the start of therapy with the claimed agent, a CT scan of the lungs was performed. The conclusion based on the results of the examination is "healthy". No signs of inflammation or residual manifestations were found. The control PCR test was negative.

**Example 10.** Patient R., aged 46, was diagnosed with COVID-19 (PCR method).

Clinical signs at the start of treatment: body temperature 39.6°C, bilateral lung damage (20% and 30%, respectively) according to CT, dry cough, retrosternal pain, general weakness, sweating, constant headache, does not smell.

Treatment with the claimed agent was started on the 5^{th} day of the disease. The agent was administered in 5 mL daily for 7 days with an interval of 24 h. Any antibiotics were no longer used.

### Result.

On the 2^{nd} day from the beginning of treatment with the claimed agent, the cough intensified and passed into the wet stage. The temperature dropped to 37.0°C, the headache disappeared. On the 3^{rd} day from the start of therapy, the temperature returned to normal (36.6°C), any pain symptoms disappeared. The general condition has improved. The patient noted the active discharge of sputum from his lungs, a surge of strength, and the absence of sweating. On the 7^{th} day, objectively, the patient recorded a complete recovery.

On the 14^{th} day after the start of therapy with the claimed agent, a CT scan of the lungs was performed. The conclusion based on the results of the examination is "healthy". No signs of inflammation or residual manifestations were found. The control PCR test was negative.

Thus, the agent has an intracellular antiseptic effect, activates the production of endogenous formic aldehyde in humans and animals in the absence of side effects and toxic effects.

## Claims

1. The use of the immunomodulating agent containing formic aldehyde in an amount of 0.073- 0.075% in isotonic sodium chloride solution as an agent for intramuscular injections with a single dose of 5 mL per injection for the treatment of coronavirus, retroviral infections and hepatitis C.

2. The use of the agent according to claim 1 by administering it once a day for seven days.

3. The use of the agent according to claim 1 by introducing it twice, when the second time is either on the 7^{th} day after the first injection or a month after the first injection.

4. The use of the agent according to claim 1 by introducing it on the 2^{nd}, 3^{rd} and 10^{th} day after the first injection.

5. The use of the agent according to claim 1 by introducing it 3 times with an interval between each injection of 12 hours, then after one or two days another 5 times with an interval between each injection of 12 hours, then, a day after the last injection, a single injection is carried out, then after three days a repeated single injection is carried out, and the final single administration of the agent is carried out five days after the last injection.
